# EUROPEAN PATENT APPLICATION

(11) **EP 4 837 559 A1**
(43) Date of publication of application: **11.11.2026**
(21) Application number: 25803348.9
(22) Date of filing: 15.04.2025
(51) Int. Cl.: A61K 8/19, A61K 8/25, A61K 8/27, A61K 8/29, A61K 8/37, A61K 8/60, A61Q 1/00

(54) **POWDER MATERIAL FOR COSMETIC, METHOD FOR PRODUCING SAME, AND COSMETIC**

(30) Priority: 14.05.2024 JP 2024078399
(71) Applicant: Daito Kasei Kogyo Co., Ltd., Osaka-shi, Osaka 535-0005 (JP)
(72) Inventor: UKIMI Masaya, Osaka-shi, Osaka 535-0005 (JP); YAMAGUCHI Natsumi, Osaka-shi, Osaka 535-0005 (JP); HAKUTO Maki, Osaka-shi, Osaka 535-0005 (JP); OHTA Atsuko, Osaka-shi, Osaka 535-0005 (JP); DOI Nanako, Osaka-shi, Osaka 535-0005 (JP); UMAKOSHI Chiaki, Osaka-shi, Osaka 535-0005 (JP)
(74) Representative: Cabinet Beau de Loménie
(86) International application number: PCT/JP2025/014801
(87) International publication number: WO 2025/239097

(57) **Abstract**

Provided is a powder material for a cosmetic, comprising a powder for a cosmetic surface-treated with a surface treatment agent. The surface treatment agent contains a component (a): a mannosylerythritol lipid represented by formula (1), and a component (b): an ester compound of glycerol and a fatty acid having 8 to 20 carbon atoms. The content of the component (a) is 0.5 to 3 mass%. The content of the component (b) is 0.1 to 10 mass%.

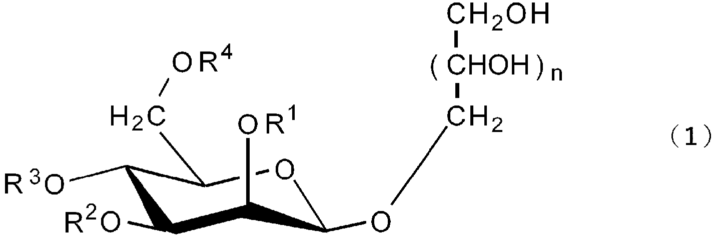

## Description

### TECHNICAL FIELD

The present invention relates to a powder material for a cosmetic that contains a powder for a cosmetic which is surface-treated with a surface treatment agent, a method for producing the powder material for a cosmetic, and a cosmetic containing the powder material for a cosmetic.

### BACKGROUND ART

Cosmetics contain various powders for a cosmetic such as inorganic powders and organic powders. Powder materials for a cosmetic have been proposed which are obtained by surface-treating (covering) the surfaces of these powders for a cosmetic with various surface treatment agents, depending on the aims of containing the powders.

For example, a powder material for a cosmetic that is covered with an ester compound of glycerol and a fatty acid having 8 to 20 carbon atoms has been proposed (see Patent Document 1). It is considered that this powder material for a cosmetic has high water resistance and good dispersibility in any of hydrocarbon oil agents, ester oil agents, and silicone oil agents.

Meanwhile, for example, a powder material for a cosmetic has been proposed in which, in order to improve feel of pigment, the surface of a hydrophobic powder such as a pigment for a cosmetic is covered with a surface treatment agent containing a mannosylerythritol lipid, which is a glycolipid produced by yeast or the like using olive oil or the like as a raw material (see Patent Documents 2 and 3). It is considered that water-in-oil type foundations containing these powder materials for a cosmetic have an excellent barrier function and moisture retaining performance for the skin, thereby providing fresh and dewy texture to the skin.

### CITATION LIST

### PATENT LITERATURE

Patent Document 1: International Publication WO2022/030462
Patent Document 2: International Publication WO2011/040357
Patent Document 3: Japanese Unexamined Patent Application Publication No. 2017-81907

### SUMMARY OF INVENTION

### TECHNICAL PROBLEM

However, the powder material for a cosmetic of Patent Document 1, in which a powder for a cosmetic is surface-treated with an ester compound of glycerol and a fatty acid having 8 to 20 carbon atoms, is likely to have less water resistance and dispersibility in an oil agent. The powder materials for a cosmetic of Patent Documents 2 and 3, in which a powder for a cosmetic is surface-treated with a mannosylerythritol lipid, are likely to have less emulsion stability.

With the above circumstances in mind, the present invention has been made. It is an object of the present invention to provide a powder material for a cosmetic that has excellent water resistance, dispersibility in an oil agent, and emulsion stability, a method for producing the powder material for a cosmetic, and a cosmetic containing the powder material for a cosmetic.

### SOLUTION TO PROBLEM

To attain the above object, a characteristic feature of a powder material for a cosmetic according to the present invention is a powder material for a cosmetic comprising a powder for a cosmetic surface-treated with a surface treatment agent, wherein
the surface treatment agent contains
   a component (a): a mannosylerythritol lipid represented by formula (1) described below, and
   a component (b): an ester compound of glycerol and a fatty acid having 8 to 20 carbon atoms,
the content of the component (a) is 0.5 to 3 mass%, and
the content of the component (b) is 0.1 to 10 mass%. 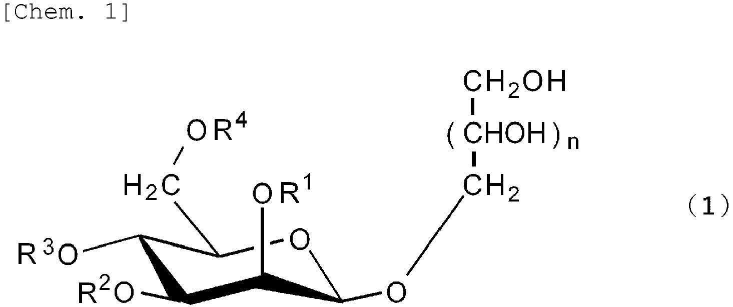
(in the formula (1), R¹ and R² each independently represent an aliphatic acyl group having 6 to 20 carbon atoms, R³ and R⁴ each independently represent a hydrogen atom or an acetyl group, and n represents an integer of 2 to 4)

With the powder material for a cosmetic having the above feature, the powder for a cosmetic is surface-treated with the surface treatment agent containing the component (a) and the component (b) such that the contents of the component (a) and the component (b) are in the above appropriate ranges. As a result, excellent water resistance, dispersibility in an oil agent, and emulsion stability are achieved. In addition, because the contents of the component (a) and the component (b) are set in the above appropriate ranges, the component (a) and the component (b) cover the surface of the powder for a cosmetic, so that the powder material for a cosmetic has powder properties as a whole. As a result, the resultant powder for a cosmetic can have further improved water resistance, dispersibility in an oil agent, and emulsion stability. Furthermore, fresh feeling of use can be obtained.

In the powder material for a cosmetic according to the present invention,
the component (b) is preferably selected from the group consisting of diglyceryl tetraisostearate, diglyceryl triisostearate, glyceryl tri(caprylate/caprate), glyceryl dilaurate, glyceryl triisostearate, decaglyceryl monoisostearate, and decaglyceryl diisostearate.

With the powder material for a cosmetic having the above feature, the above appropriate compounds are selected as the component (b). As a result, the water resistance, dispersibility in an oil agent, and emulsion stability thereof can be further improved.

In the powder material for a cosmetic according to the present invention,
preferably, R¹ and R² each independently represent an aliphatic acyl group having 10 to 14 carbon atoms.

With the powder material for a cosmetic having the above feature, the above appropriate aliphatic acyl group is selected as R¹ and R². As a result, the hydrophobicity thereof can be appropriately improved, resulting in a further improvement in the water resistance, dispersibility in an oil agent, and emulsion stability thereof.

In the powder material for a cosmetic according to the present invention,
the powder for a cosmetic is preferably at least one selected from the group consisting of titanium oxide, yellow iron oxide, red iron oxide, black iron oxide, talc, zinc oxide, silicon oxide, pearl mica, mica, and sericite.

With the powder material for a cosmetic having the above feature, the above appropriate powders are selected as the powder for a cosmetic. As a result, the water resistance, dispersibility in an oil agent, and emulsion stability thereof can be further improved.

To attain the above object, a characteristic feature of a method for producing a powder material for a cosmetic according to another aspect of the present invention, comprises
mixing a powder for a cosmetic with a liquid mixture obtained by dispersing, suspending, or dissolving, in a solvent,
   a component (a): a mannosylerythritol lipid represented by formula (1) described below, and
   a component (b): an ester compound of glycerol and a fatty acid having 8 to 20 carbon atoms, and
heat-treating the mixture obtained by the mixing. 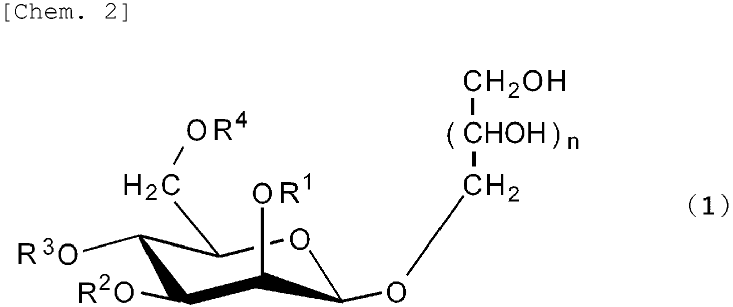
(in the formula (1), R¹ and R² each independently represent an aliphatic acyl group having 6 to 20 carbon atoms, R³ and R⁴ each independently represent a hydrogen atom or an acetyl group, and n represents an integer of 2 to 4)

With the method for producing a powder material for a cosmetic that has the above feature, the mixing step and the heat treatment step are performed. As a result, a powder material for a cosmetic that has excellent water resistance, dispersibility in an oil agent, and emulsion stability can be obtained.

In the method for producing a powder material for a cosmetic according to the present invention,
in the mixing, the mixing ratio by mass of the solvent to the component (a) contained in the liquid mixture is preferably adjusted to at least 10:1.

With the method for producing a powder material for a cosmetic that has the above feature, the mixing ratio of the solvent and the component (a) contained in the liquid mixture is adjusted in the above appropriate range. As a result, the dispersibility of the component (a) in the solvent can be improved.

In the method for producing a powder material for a cosmetic according to the present invention,
in the heat-treating, the mixture is preferably heat-treated at 70 to 150°C.

With the method for producing a powder material for a cosmetic that has the above feature, the heat treatment of the mixture is performed at the above appropriate temperature. As a result, the ester compound of a fatty acid having 8 to 20 carbon atoms and glycerol, which is the component (b), is present on the surface of the powder for a cosmetic with the hydrophilic group of the ester compound pointing toward the powder for a cosmetic, resulting in a powder material for a cosmetic having excellent water resistance. In addition, the component (a) and the component (b) can be satisfactorily fixed to the powder for a cosmetic.

In the method for producing a powder material for a cosmetic according to the present invention,
the solvent is preferably an organic solvent and/or water.

With the method for producing a powder material for a cosmetic that has the above feature, an organic solvent and/or water are used as the solvent. As a result, impurities are not left in the powder material for a cosmetic after the heat treatment step, resulting in a product having high purity.

To attain the above object, a characteristic feature of a cosmetic according to another aspect of the present invention, comprises the above powder material for a cosmetic.

The cosmetic that has the above feature comprises the above powder material for a cosmetic, and therefore, has excellent water resistance, dispersibility in an oil agent, and emulsion stability.

### BRIEF DESCRIPTION OF DRAWINGS

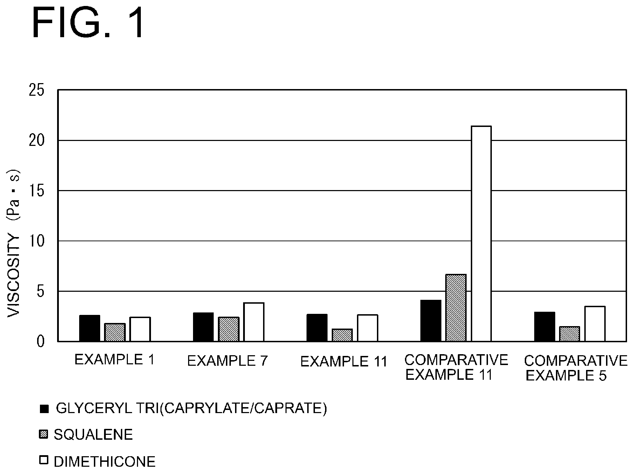

[FIG. 1] FIG. 1 is a graph showing results of an oil agent dispersibility assessment test.

### DESCRIPTION OF EMBODIMENTS

A powder material for a cosmetic according to the present invention, a method for producing the powder material for a cosmetic, and a cosmetic containing the powder material for a cosmetic will be described below in detail. It should be noted that the present invention is not intended to be limited to embodiments and examples described below.

### [Powder material for cosmetic]

A powder material for a cosmetic according to the present invention contains a powder for a cosmetic that is surface-treated with a surface treatment agent. The surface treatment agent contains a component (a) and a component (b) described below.

### <Component (a): mannosylerythritol lipid>

The component (a) is a mannosylerythritol lipid (hereinafter also referred to as a "MEL") represented by formula (1) described below. MELs are a collective name for glycolipids composed of mannose, a sugar alcohol, and fatty acids. More specifically, a MEL has a glycolipid obtained by formation of a glycosidic bond between the hydroxy group at the 1-position of mannose and the sugar alcohol through dehydration condensation, and formation of an ester bond between the hydroxy groups at the 2- and 3-positions of mannose and the fatty acids through dehydration condensation. In the MEL of the present invention, the hydrogen atoms of the hydroxy groups at the 4- and 6-positions of mannose are unsubstituted (R³ and R⁴ are a hydrogen atom), or at least one thereof is substituted with an acetyl group (at least one of R³ and R⁴ is an acetyl group).

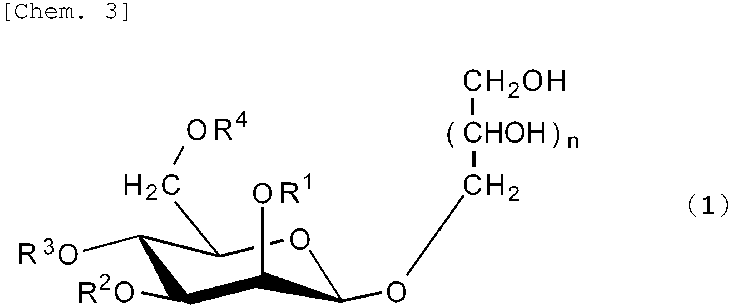

In formula (1), R¹ and R² each independently represent an aliphatic acyl group having 6 to 20 carbon atoms, R³ and R⁴ each independently represent a hydrogen atom or an acetyl group, and n represents an integer of 2 to 4.

The structure of the MEL represented by formula (1) may vary depending on the presence or absence of an acetyl group (Ac) added to the mannose residue as the substituents R³ and R⁴, the type of a sugar alcohol joined to mannose by a glycosidic bond, and the isomer of the sugar alcohol.

For example, it is preferable that R¹ and R² each independently be an aliphatic acyl group having 10 to 14 carbon atoms. In the case in which the above appropriate aliphatic acyl groups are selected as R¹ and R², hydrophobicity can be appropriately increased, so that the water resistance, dispersibility in an oil agent, and emulsion stability of the powder material for a cosmetic can be further improved.

For example, embodiments of R³ and R⁴ include an embodiment in which R³ and R⁴ are an acetyl group (see MEL-A described below), an embodiment in which R³ is a hydrogen atom and R⁴ is an acetyl group (see MEL-B and inverted MEL-B described below), an embodiment in which R³ is an acetyl group and R⁴ is a hydrogen atom (see MEL-C described below), and an embodiment in which R³ and R⁴ are a hydrogen atom (see MEL-D and inverted MEL-D described below). Even in the case in which any of these embodiments is selected, the water resistance, dispersibility in an oil agent, and emulsion stability of the powder material for a cosmetic can be similarly improved.

It is preferable that n be an integer of 2 or 3, more preferably 2. In the case in which the above appropriate integer is selected as n, hydrophilicity can be appropriately reduced, so that the the water resistance, dispersibility in an oil agent, and emulsion stability of the powder material for a cosmetic can be further improved.

Specifically, examples of the MEL include MEL-A that has a structure represented by formula (2) shown below, MEL-B that has a structure represented by formula (3) or (4) shown below, MEL-C that has a structure represented by formula (5) shown below, and MEL-D that has a structure represented by formula (6) or (7) shown below. MEL-B represented by formula (3) and MEL-B represented by (4) are optical isomers. MEL-B having the structure represented by formula (4), which is an optical isomer with respect to MEL-B having the structure represented by formula (3), is referred to as inverted MEL-B. MEL-D represented by formula (6) and MEL-D represented by formula (7) are optical isomers. MEL-D having the structure represented by formula (7), which is an optical isomer with respect to MEL-D having the structure represented by formula (6), is referred to as inverted MEL-D.

Of them, as the MEL, MEL-A, having the structure represented by formula (2), that is produced by the microorganism Pseudozyma antarctica, and MEL-B (inverted type), having the structure represented by formula (4), that is produced by the microorganism Pseudozyma tsukubaensis, are preferable. In the case in which as the MEL, one having the above appropriate structure is selected, the water resistance, dispersibility in an oil agent, and emulsion stability of the powder material for a cosmetic can be further improved. The above MELs may be used alone or in combination.

It should be noted that the structures represented by formulas (2), (3), (5), and (6) are 4-O-β-D-mannopyranosyl-(2S,3R)-erythritol structures. Meanwhile, the term "inverted" used for the structures represented by formulas (4) and (7) below is used in order to distinguish MELs that are optical isomers from each other. As used herein, the term "inverted" indicates compounds having 4-O-β-D-mannopyranosyl-meso(2R,3S)-erythritol structures shown in formulas (4) and (7) below.

### (MEL-A)

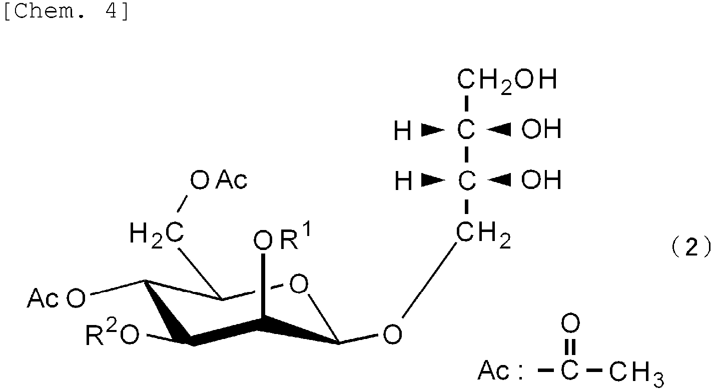

### (MEL-B)

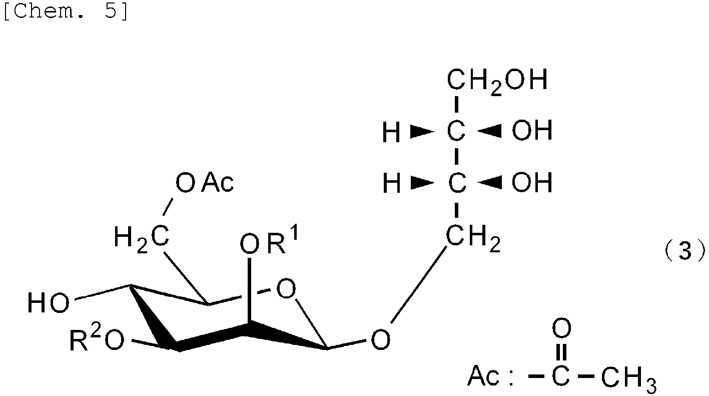

### (Inverted MEL-B)

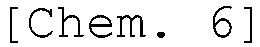

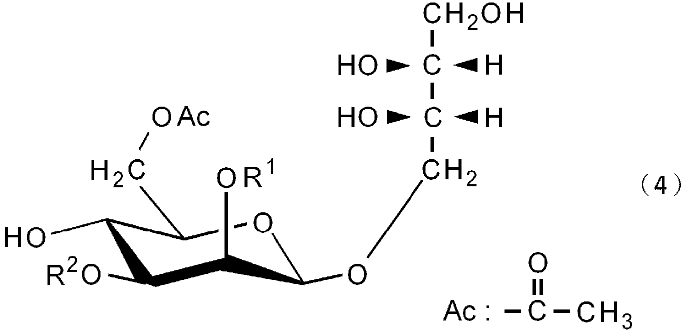

### (MEL-C)

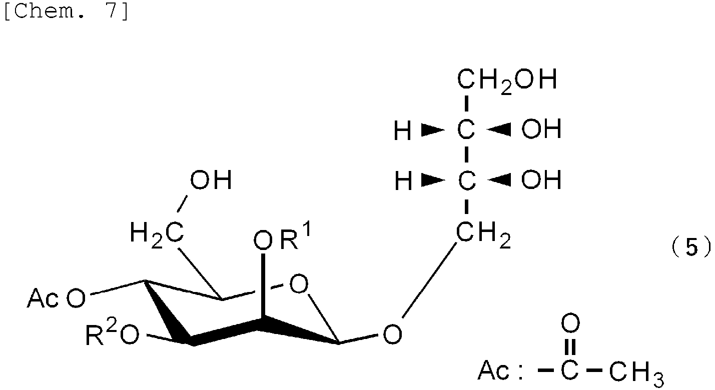

### (MEL-D)

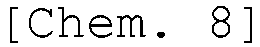

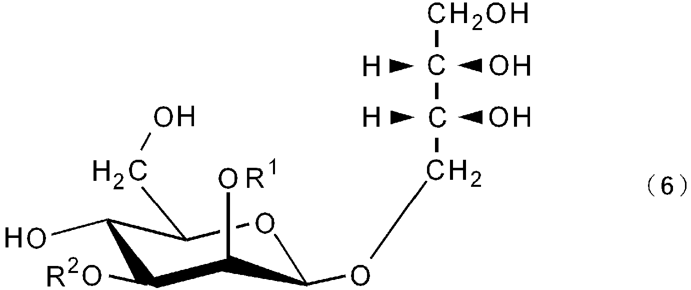

### (Inverted MEL-D)

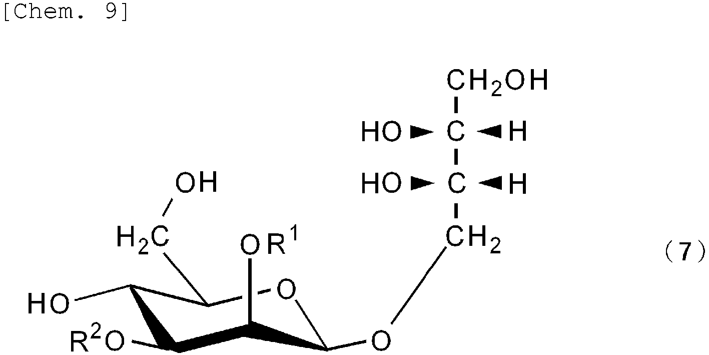

In formulas (2) to (7), R¹ and R² each independently represent an aliphatic acyl group having 6 to 20 carbon atoms. Similarly to the foregoing description, it is preferable that R¹ and R² each independently be an aliphatic acyl group having 10 to 14 carbon atoms.

The content of the component (a) in the powder material for a cosmetic is 0.5 to 3 mass%. In the case in which the content of the component (a) is set in the above appropriate range, the water resistance, dispersibility in an oil agent, and emulsion stability of the powder material for a cosmetic can be further improved.

### <Component (b): ester compound of glycerol and fatty acid having 8 to 20 carbon atoms>

In the case in which the powder for a cosmetic is surface-treated with the component (b), which is an ester compound of glycerol and a fatty acid having 8 to 20 carbon atoms, together with the component (a), the component (b) and the component (a) can form a coating film, in which the component (b) and the component (a) form a composite.

The component (b) is preferably diglyceryl tetraisostearate, diglyceryl triisostearate, glyceryl tri(caprylate/caprate), glyceryl dilaurate, glyceryl triisostearate, decaglyceryl monoisostearate, or decaglyceryl diisostearate. In the case in which the above appropriate compounds are selected as the component (b), water resistance, dispersibility in an oil agent, and emulsion stability can be further improved. The components (b) may be used alone or in combination.

The content of the component (b) in the powder material for a cosmetic is 0.1 to 10 mass%. In the case in which the content of the component (b) is set in the above appropriate range, the water resistance, dispersibility in an oil agent, and emulsion stability of the powder material for a cosmetic can be further improved. In addition, in the case in which the content of the component (a) in the powder material for a cosmetic is set to 0.5 to 3 mass% and the content of the component (b) is set in the above appropriate range (0.1 to 10 mass%), the component (a) and the component (b) cover the surface of the powder for a cosmetic, and the powder material for a cosmetic has powder properties as a whole. As a result, the water resistance, dispersibility in an oil agent, and emulsion stability of the resultant powder for a cosmetic can be further improved. Furthermore, fresh feeling of use can be provided.

### <Powder for cosmetic>

The powder for a cosmetic is a powder that is surface-treated (covered) with a surface treatment agent. In this respect, the powder for a cosmetic can serve as a base for a powder material for a cosmetic. As the powder for a cosmetic, conventionally known powders applicable to cosmetics are available. In addition, various powders can be used irrespective of shape, particle size, particle structure, and the like. Examples of the powder for a cosmetic include inorganic powders, organic powders, surfactant metal salt powders, colored pigments, pearl pigments, and metal powder pigments.

Examples of the inorganic powders include titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, muscovite, synthetic mica, phlogopite, lepidolite, biotite, lithium mica, silicate, anhydride silicate, aluminum silicate, magnesium silicate, aluminum magnesium silicate, calcium silicate, barium silicate, strontium silicate, metal salts of tungstic acid, hydroxyapatite, vermiculite, higilite, bentonite, montmorillonite, hectorite, zeolite, ceramic powders, calcium secondary phosphate, alumina, aluminum hydroxide, boron nitride, and silica.

Examples of the organic powders include polyamide powders, polyester powders, polyethylene powders, polypropylene powders, polystyrene powders, polyurethane powders, benzoguanamine powders, polymethylbenzoguanamine powders, polytetrafluoroethylene powders, polymethylmethacrylate powders, cellulose, silk powders, nylon powders, nylon 12, nylon 6, acrylic powders, acrylic elastomers, styrene-acrylic acid copolymers, divinylbenzene-styrene copolymers, vinyl resins, urea resins, phenolic resins, fluorine resins, silicon resins, acrylic resins, melamine resins, epoxy resins, polycarbonate resins, microcrystalline fiber powders, starch powders, and lauroyl lysine.

Examples of the surfactant metal salt powders (metallic soaps) include zinc stearate, aluminum stearate, calcium stearate, magnesium stearate, zinc myristate, magnesium myristate, zinc cetyl phosphate, calcium cetyl phosphate, and zinc sodium cetyl phosphate.

Examples of the colored pigments include inorganic red pigments such as iron oxides (e.g., iron(III) oxide (red iron oxide)), iron hydroxide, and iron titanate, inorganic brown pigments such as γ-iron oxide, inorganic yellow pigments such as yellow oxide of iron (yellow iron oxide) and loess, inorganic black pigments such as iron(II,III) oxide (black iron oxide) and carbon black, inorganic violet pigments such as manganese violet and cobalt violet, inorganic green pigments such as chromium hydroxide, chromium oxide, cobalt oxide, and cobalt titanate, inorganic blue pigments such as iron blue and ultramarine blue, microparticle powders such as microparticle titanium oxide, microparticle cerium oxide, and microparticle zinc oxide, laked tar-based colored elements, laked natural colored elements, and synthetic resin powders that are composites of these powders.

Examples of the pearl pigments include pearl micas such as titanium oxide-covered mica, bismuth oxychloride, titanium oxide-covered bismuth oxychloride, titanium oxide-covered talc, fish scale flake, and titanium oxide-covered colored mica.

Examples of the metal powder pigments include aluminum powder, copper powder, and stainless powder.

Of the above powders for a cosmetic, titanium oxide, yellow iron oxide, red iron oxide, black iron oxide, talc, zinc oxide, silicon oxide, pearl mica, mica, and sericite are preferable. In the case in which the above appropriate powders are selected as the powder for a cosmetic, water resistance, dispersibility in an oil agent, and emulsion stability can be further improved. The powders for a cosmetic may be used alone or in combination.

### <Other components>

The powder material for a cosmetic according to the present invention can contain other components in addition to the powder for a cosmetic, the component (a), and the component (b). Examples of the other components include antimicrobial agents, antiviral agents, microbicides, deodorants, antioxidants, stabilizers, and pH adjusting agents.

### <Method for producing powder material for cosmetic>

The powder material for a cosmetic according to the present invention can be produced by carrying out a mixing step and a heat treatment step described below.

### (Mixing step)

In the mixing step, a liquid mixture obtained by dispersing, suspending, or dissolving the component (a) and the component (b) in a solvent is mixed with the powder for a cosmetic.

The solvent used in the mixing step is not particularly limited as long as the component (a) and the component (b) can be dispersed, suspended, or dissolved in the solvent. For example, organic solvents and/or water are preferable. In the case in which an organic solvent and/or water are used as the solvent, impurities are not left in the powder material for a cosmetic after the heat treatment step, resulting in a product having high purity.

Examples of the organic solvents include alcoholic organic solvents such as ethanol, isopropylalcohol, and isobutanol, hydrocarbon organic solvents such as toluene, n-hexane, and cyclohexane, and polar organic solvents such as acetone, ethyl acetate, and butyl acetate.

In the mixing step, the mixing ratio by mass of the solvent to the component (a) in the liquid mixture is preferably adjusted to at least 10:1 (the mass of the solvent is at least 10 times as great as that of the component (a)). Specifically, for example, in the case in which water is used as the solvent, the mixing ratio by mass of the solvent to the component (a) is preferably adjusted to at least 15:1 (the mass of the solvent is at least 15 times as great as that of the component (a)). In the case in which an organic solvent is used as the solvent, the mixing ratio by mass of the solvent to the component (a) is preferably adjusted to at least 10:1 (the mass of the solvent is at least 10 times as great as that of the component (a)). In the case in which the mixing ratio of the solvent to the component (a) in the liquid mixture is adjusted in the above appropriate range, the dispersibility of the component (a) in the solvent can be improved.

In the mixing step, in the case in which water is used as the solvent, the component (a) and the component (b) are preferably dispersed, suspended, or dissolved in water of 50 to 60°C. In the case in which an organic solvent is used as the solvent, the component (a) and the component (b) are preferably dispersed, suspended, or dissolved in water of 40 to 60°C. In the case in which the mixing temperature is set in the above appropriate ranges, depending on the type of the solvent, the component (a) and the component (b) can be more uniformly dispersed, suspended, or dissolved in each solvent.

In order to mix the liquid mixture and the powder for a cosmetic, an appropriate mixing means can be selected, depending on the concentration (added amount), viscosity, or the like of the solvent. Examples of the mixing means include mixers such as a disper, Henschel mixer, loedige mixer, kneader, V-type mixer, roll mill, bead mill, and twin-screw kneader. In mixing using such a mixer, the liquid mixture is preferably gradually added to the powder for a cosmetic while stirring the powder for a cosmetic. Such a mixing technique can cause the liquid mixture to more uniformly adhere to the surface of the powder for a cosmetic.

### (Heat treatment step)

In the heat treatment step, the mixture obtained in the mixing step is subjected to a heat treatment. By performing the heat treatment step, a powder material for a cosmetic is obtained in which the surface of the powder for a cosmetic is surface-treated (covered) with the surface treatment agent containing the component (a) and the component (b).

In the heat treatment step, the mixture is preferably subjected to a heat treatment at 70 to 150°C. By performing the heat treatment of the mixture at the above appropriate temperature, the ester compound of a fatty acid having 8 to 20 carbon atoms and glycerol, which is the component (b), is present on the surface of the powder for a cosmetic with the hydrophilic group of the ester compound pointing toward the powder for a cosmetic, resulting in a powder material for a cosmetic having excellent water resistance.

The heat treatment of the mixture can be performed using an electric furnace, chamber furnace, kiln, infrared heater, or the like. Although the heat treatment is typically performed in the presence of air, the heat treatment may be performed under vacuum or in an inert gas atmosphere in the case in which the mixture contains a material that is easily oxidized.

Pulverization is preferably carried out after the heat treatment. In the case in which pulverization is carried out after the heat treatment, a commonly used pulverizer such as a hammer mill, ball mill, sand mill, or jet mill can be used. Every pulverlizer can provide similar quality, and therefore, the pulverizer is not particularly limited.

### <Cosmetic>

A cosmetic according to the present invention contains the above powder material for a cosmetic. The content of the powder material for a cosmetic in the cosmetic is not particularly limited, and is preferably 0.1 to 95 mass%, for example. In the case in which the content of the powder material for a cosmetic is set in the above appropriate range, the powder material for a cosmetic can sufficiently exhibit the above effect.

The cosmetic according to the present invention is either an oil-in-water type (O/W type) emulsion or a water-in-oil type (W/O type) emulsion. However, a water-in-oil type (W/O type) emulsion is preferable.

The cosmetic according to the present invention can contain components commonly used in cosmetics, such as powders other than the powder material for a cosmetic, surfactants, oil agents, gelling agents, polymers, cosmetic components, moisturizers, colored elements, antiseptics, and aroma chemicals, in a range that does not impair the effect of the present invention.

### [Examples]

Powder materials for a cosmetic according to the present invention (Examples 1 to 14) were formulated and subjected to an oil agent dispersibility assessment test, water repellency (water resistance) test, and emulsion stability assessment. For comparison, powder materials for a cosmetic (Comparative Examples 1 to 12) that depart from the range of the present invention were formulated and subjected to a similar test and assessment.

### [Example 1]

To 12.5 g of isopropanol of 40°C, added were 1.0 g of a mannosylerythritol lipid (MEL-A, R¹ and R²: an aliphatic acyl group having 10 to 14 carbon atoms, R³ and R⁴: an acetyl group) and 0.5 g of diglyceryl tetraisostearate (DG4ISA), which served as surface treatment agents, followed by dispersion, to prepare a liquid mixture. Next, the liquid mixture was dropped to and mixed with 98.5 g of titanium oxide, which served as a powder for a cosmetic, for 20 minutes while the titanium oxide was being stirred using a mixer, to prepare a mixture. The mixture was subjected to a heat treatment at 110°C for 6 hours. The resultant dry powder was pulverized using a hammer mill to obtain a powder material for a cosmetic according to Example 1. The total content of MEL-A and DG4ISA in the powder material for a cosmetic of Example 1 was 1.5 mass%.

### [Example 2]

To 25.0 g of isopropanol of 40°C, added were 1.5 g of MEL-A and 0.5 g of DG4ISA, which served as surface treatment agents, followed by dispersion, to prepare a liquid mixture. Next, the liquid mixture was dropped to and mixed with 98.0 g of yellow iron oxide, which served as a powder for a cosmetic, for 20 minutes while the yellow iron oxide was being stirred using a mixer, to prepare a mixture. The mixture was subjected to a heat treatment at 110°C for 6 hours. The resultant dry powder was pulverized using a hammer mill to obtain a powder material for a cosmetic according to Example 2. The total content of MEL-A and DG4ISA in the powder material for a cosmetic of Example 2 was 2.0 mass%.

### [Example 3]

To 25.0 g of isopropanol of 40°C, added were 1.0 g of MEL-A and 0.5 g of DG4ISA, which served as surface treatment agents, followed by dispersion, to prepare a liquid mixture. Next, the liquid mixture was dropped to and mixed with 98.5 g of red iron oxide, which served as a powder for a cosmetic, for 20 minutes while the red iron oxide was being stirred using a mixer, to prepare a mixture. The mixture was subjected to a heat treatment at 110°C for 6 hours. The resultant dry powder was pulverized using a hammer mill to obtain a powder material for a cosmetic according to Example 3. The total content of MEL-A and DG4ISA in the powder material for a cosmetic of Example 3 was 1.5 mass%.

### [Example 4]

To 12.5 g of isopropanol of 40°C, added were 1.0 g of MEL-A and 0.5 g of DG4ISA, which served as surface treatment agents, followed by dispersion, to prepare a liquid mixture. Next, the liquid mixture was dropped to and mixed with 98.5 g of black iron oxide, which served as a powder for a cosmetic, for 20 minutes while the black iron oxide was being stirred using a mixer, to prepare a mixture. The mixture was subjected to a heat treatment at 110°C for 6 hours. The resultant dry powder was pulverized using a hammer mill to obtain a powder material for a cosmetic according to Example 4. The total content of MEL-A and DG4ISA in the powder material for a cosmetic of Example 4 was 1.5 mass%.

### [Example 5]

To 12.5 g of isopropanol of 40°C, added were 0.5 g of MEL-A and 0.1 g of DG4ISA, which served as surface treatment agents, followed by dispersion, to prepare a liquid mixture. Next, the liquid mixture was dropped to and mixed with 99.4 g of titanium oxide, which served as a powder for a cosmetic, for 20 minutes while the titanium oxide was being stirred using a mixer, to prepare a mixture. The mixture was subjected to a heat treatment at 110°C for 6 hours. The resultant dry powder was pulverized using a hammer mill to obtain a powder material for a cosmetic according to Example 5. The total content of MEL-A and DG4ISA in the powder material for a cosmetic of Example 5 was 0.6 mass%.

### [Example 6]

To 12.5 g of isopropanol of 40°C, added were 3.0 g of MEL-A and 10.0 g of DG4ISA, which served as surface treatment agents, followed by dispersion, to prepare a liquid mixture. Next, the liquid mixture was dropped to and mixed with 87.0 g of titanium oxide, which served as a powder for a cosmetic, for 20 minutes while the titanium oxide was being stirred using a mixer, to prepare a mixture. The mixture was subjected to a heat treatment at 110°C for 6 hours. The resultant dry powder was pulverized using a hammer mill to obtain a powder material for a cosmetic according to Example 6. The total content of MEL-A and DG4ISA in the powder material for a cosmetic of Example 6 was 13.0 mass%.

### [Example 7]

To 15.0 g of water of 50°C, added were 1.0 g of MEL-A and 0.5 g of DG4ISA, which served as surface treatment agents, followed by dispersion, to prepare a liquid mixture. Next, the liquid mixture was dropped to and mixed with 98.5 g of titanium oxide, which served as a powder for a cosmetic, for 20 minutes while the titanium oxide was being stirred using a mixer, to prepare a mixture. The mixture was subjected to a heat treatment at 110°C for 6 hours. The resultant dry powder was pulverized using a hammer mill to obtain a powder material for a cosmetic according to Example 7. The total content of MEL-A and DG4ISA in the powder material for a cosmetic of Example 7 was 1.5 mass%.

### [Example 8]

To 25.0 g of water of 50°C, added were 1.5 g of MEL-A and 0.5 g of DG4ISA, which served as surface treatment agents, followed by dispersion, to prepare a liquid mixture. Next, the liquid mixture was dropped to and mixed with 98.0 g of yellow iron oxide, which served as a powder for a cosmetic, for 20 minutes while the yellow iron oxide was being stirred using a mixer, to prepare a mixture. The mixture was subjected to a heat treatment at 110°C for 6 hours. The resultant dry powder was pulverized using a hammer mill to obtain a powder material for a cosmetic according to Example 8. The total content of MEL-A and DG4ISA in the powder material for a cosmetic of Example 8 was 2.0 mass%.

### [Example 9]

To 15.0 g of water of 50°C, added were 1.0 g of MEL-A and 0.5 g of DG4ISA, which served as surface treatment agents, followed by dispersion, to prepare a liquid mixture. Next, the liquid mixture was dropped to and mixed with 98.5 g of red iron oxide, which served as a powder for a cosmetic, for 20 minutes while the red iron oxide was being stirred using a mixer, to prepare a mixture. The mixture was subjected to a heat treatment at 110°C for 6 hours. The resultant dry powder was pulverized using a hammer mill to obtain a powder material for a cosmetic according to Example 9. The total content of MEL-A and DG4ISA in the powder material for a cosmetic of Example 9 was 1.5 mass%.

### [Example 10]

To 15.0 g of water of 50°C, added were 1.0 g of MEL-A and 0.5 g of DG4ISA, which served as surface treatment agents, followed by dispersion, to prepare a liquid mixture. Next, the liquid mixture was dropped to and mixed with 98.5 g of black iron oxide, which served as a powder for a cosmetic, for 20 minutes while the black iron oxide was being stirred using a mixer, to prepare a mixture. The mixture was subjected to a heat treatment at 110°C for 6 hours. The resultant dry powder was pulverized using a hammer mill to obtain a powder material for a cosmetic according to Example 10. The total content of MEL-A and DG4ISA in the powder material for a cosmetic of Example 10 was 1.5 mass%.

### [Example 11]

To 15.0 g of water of 50°C, added were 1.0 g of inverted MEL-B (R¹ and R²: an aliphatic acyl group having 10 to 14 carbon atoms, R³: a hydrogen atom, R⁴: an acetyl group) and 0.5 g of DG4ISA, which served as surface treatment agents, followed by dispersion, to prepare a liquid mixture. Next, the liquid mixture was dropped to and mixed with 98.5 g of titanium oxide, which served as a powder for a cosmetic, for 20 minutes while the titanium oxide was being stirred using a mixer, to prepare a mixture. The mixture was subjected to a heat treatment at 110°C for 6 hours. The resultant dry powder was pulverized using a hammer mill to obtain a powder material for a cosmetic according to Example 11. The total content of inverted MEL-B and DG4ISA in the powder material for a cosmetic of Example 11 was 1.5 mass%.

### [Example 12]

To 15.0 g of water of 50°C, added were 1.5 g of inverted MEL-B and 0.5 g of DG4ISA, which served as surface treatment agents, followed by dispersion, to prepare a liquid mixture. Next, the liquid mixture was dropped to and mixed with 98.0 g of yellow iron oxide, which served as a powder for a cosmetic, for 20 minutes while the yellow iron oxide was being stirred using a mixer, to prepare a mixture. The mixture was subjected to a heat treatment at 110°C for 6 hours. The resultant dry powder was pulverized using a hammer mill to obtain a powder material for a cosmetic according to Example 12. The total content of inverted MEL-B and DG4ISA in the powder material for a cosmetic of Example 12 was 2.0 mass%.

### [Example 13]

To 15.0 g of water of 50°C, added were 1.0 g of inverted MEL-B and 0.5 g of DG4ISA, which served as surface treatment agents, followed by dispersion, to prepare a liquid mixture. Next, the liquid mixture was dropped to and mixed with 98.5 g of red iron oxide, which served as a powder for a cosmetic, for 20 minutes while the red iron oxide was being stirred using a mixer, to prepare a mixture. The mixture was subjected to a heat treatment at 110°C for 6 hours. The resultant dry powder was pulverized using a hammer mill to obtain a powder material for a cosmetic according to Example 13. The total content of inverted MEL-B and DG4ISA in the powder material for a cosmetic of Example 13 was 1.5 mass%.

### [Example 14]

To 15.0 g of water of 50°C, added were 1.0 g of inverted MEL-B and 0.5 g of DG4ISA, which served as surface treatment agents, followed by dispersion, to prepare a liquid mixture. Next, the liquid mixture was dropped to and mixed with 98.5 g of black iron oxide, which served as a powder for a cosmetic, for 20 minutes while the black iron oxide was being stirred using a mixer, to prepare a mixture. The mixture was subjected to a heat treatment at 110°C for 6 hours. The resultant dry powder was pulverized using a hammer mill to obtain a powder material for a cosmetic according to Example 14. The total content of inverted MEL-B and DG4ISA in the powder material for a cosmetic of Example 14 was 1.5 mass%.

### [Comparative Example 1]

A powder material for a cosmetic according to Comparative Example 1 was prepared in the same manner as that of Example 1, except for using 1.0 g of MEL-A as a surface treatment agent and 99.0 g of titanium oxide as a powder for a cosmetic. The content of MEL-A in the powder material for a cosmetic of Comparative Example 1 was 1.0 mass%.

### [Comparative Example 2]

A powder material for a cosmetic according to Comparative Example 2 was prepared in the same manner as that of Example 2, except for using 2.0 g of MEL-A as a surface treatment agent and 98.0 g of yellow iron oxide as a powder for a cosmetic. The content of MEL-A in the powder material for a cosmetic of Comparative Example 2 was 2.0 mass%.

### [Comparative Example 3]

A powder material for a cosmetic according to Comparative Example 3 was prepared in the same manner as that of Example 3, except for using 1.0 g of MEL-A as a surface treatment agent and 99.0 g of red iron oxide as a powder for a cosmetic. The content of MEL-A in the powder material for a cosmetic of Comparative Example 3 was 1.0 mass%.

### [Comparative Example 4]

A powder material for a cosmetic according to Comparative Example 4 was prepared in the same manner as that of Example 4, except for using 1.0 g of MEL-A as a surface treatment agent and 99.0 g of black iron oxide as a powder for a cosmetic. The content of MEL-A in the powder material for a cosmetic of Comparative Example 4 was 1.0 mass%.

### [Comparative Example 5]

A powder material for a cosmetic according to Comparative Example 5 was prepared in the same manner as that of Example 1, except for using 2.0 g of DG4ISA as a surface treatment agent and 98.0 g of titanium oxide as a powder for a cosmetic. The content of DG4ISA in the powder material for a cosmetic of Comparative Example 5 was 2.0 mass%.

### [Comparative Example 6]

A powder material for a cosmetic according to Comparative Example 6 was prepared in the same manner as that of Example 2, except for using 2.0 g of DG4ISA as a surface treatment agent and 98.0 g of yellow iron oxide as a powder for a cosmetic. The content of DG4ISA in the powder material for a cosmetic of Comparative Example 6 was 2.0 mass%.

### [Comparative Example 7]

A powder material for a cosmetic according to Comparative Example 7 was prepared in the same manner as that of Example 3, except for using 2.0 g of DG4ISA as a surface treatment agent and 98.0 g of red iron oxide as a powder for a cosmetic. The content of DG4ISA in the powder material for a cosmetic of Comparative Example 7 was 2.0 mass%.

### [Comparative Example 8]

A powder material for a cosmetic according to Comparative Example 8 was prepared in the same manner as that of Example 4, except for using 2.0 g of DG4ISA as a surface treatment agent and 98.0 g of black iron oxide as a powder for a cosmetic. The content of DG4ISA in the powder material for a cosmetic of Comparative Example 8 was 2.0 mass%.

### [Comparative Example 9]

A powder of titanium oxide was used as a powder for a cosmetic. The titanium oxide powder was directly used as a powder material for a cosmetic according to Comparative Example 9 without being surface-treated.

### [Comparative Example 10]

To 12.5 g of isopropanol of 40°C, added were 0.3 g of MEL-A and 0.1 g of DG4ISA, which served as surface treatment agents, followed by dispersion, to prepare a liquid mixture. Next, the liquid mixture was dropped to and mixed with 99.6 g of titanium oxide, which served as a powder for a cosmetic, for 20 minutes while the titanium oxide was being stirred using a mixer, to prepare a mixture. The mixture was subjected to a heat treatment at 110°C for 6 hours. The resultant dry powder was pulverized using a hammer mill to obtain a powder material for a cosmetic according to Comparative Example 10. The total content of MEL-A and DG4ISA in the powder material for a cosmetic of Comparative Example 10 was 0.4 mass%.

### [Comparative Example 11]

To 12.5 g of isopropanol of 40°C, added were 4.0 g of MEL-A and 10.0 g of DG4ISA, which served as surface treatment agents, followed by dispersion, to prepare a liquid mixture. Next, the liquid mixture was dropped to and mixed with 86.0 g of titanium oxide, which served as a powder for a cosmetic, for 20 minutes while the titanium oxide was being stirred using a mixer, to prepare a mixture. The mixture was subjected to a heat treatment at 110°C for 6 hours. The resultant dry powder was pulverized using a hammer mill to obtain a powder material for a cosmetic according to Comparative Example 11. The total content of MEL-A and DG4ISA in the powder material for a cosmetic of Comparative Example 11 was 14.0 mass%.

### [Comparative Example 12]

To 12.5 g of isopropanol of 40°C, added were 3.0 g of MEL-A and 11.0 g of DG4ISA, which served as surface treatment agents, followed by dispersion, to prepare a liquid mixture. Next, the liquid mixture was dropped to and mixed with 86.0 g of titanium oxide as a powder for a cosmetic, for 20 minutes while the titanium oxide was being stirred using a mixer, to prepare a mixture. The mixture was subjected to a heat treatment at 110°C for 6 hours. The resultant dry powder was pulverized using a hammer mill to obtain a powder material for a cosmetic according to Comparative Example 12. The total content of MEL-A and DG4ISA in the powder material for a cosmetic of Comparative Example 12 was 14.0 mass%.

### [Water repellency (water resistance) test]

The powder materials for a cosmetic of Examples 1 to 4 and 7 to 14 and Comparative Examples 1 to 9 were subjected to a water repellency test in which the contact angles thereof were measured as follows. Initially, a mold was filled with an appropriate amount of a powder material for a cosmetic, which was then pressed with a compression force of 10 MPa, to form a tablet for contact angle measurement. The tablet thus prepared was placed in a contact angle measurement device (contact angle meter: LSE-B100, manufactured by NiCK Corporation). A water droplet was dropped to the tablet using a syringe. The contact angle (°) between the water droplet and the tablet was measured. The results are shown in Table 1.

**[Table 1]**

| | Powder for cosmetic | MEL content (mass%) | DG4ISA content (mass%) | Contact angle (°) |
|---|---|---|---|---|
| Example 1 | Titanium oxide | 1.0 | 0.5 | 130 |
| Example 7 | Titanium oxide | 1.0 | 0.5 | 131 |
| Example 11 | Titanium oxide | 1.0 | 0.5 | 137 |
| Comparative Example 1 | Titanium oxide | 1.0 | - | 120 |
| Comparative Example 5 | Titanium oxide | - | 2.0 | 131 |
| Example 2 | Yellow iron oxide | 1.5 | 0.5 | 134 |
| Example 8 | Yellow iron oxide | 1.5 | 0.5 | 136 |
| Example 12 | Yellow iron oxide | 1.5 | 0.5 | 139 |
| Comparative Example 2 | Yellow iron oxide | 2.0 | - | 132 |
| Comparative Example 6 | Yellow iron oxide | - | 2.0 | 135 |
| Example 3 | Red iron oxide | 1.0 | 0.5 | 131 |
| Example 9 | Red iron oxide | 1.0 | 0.5 | 135 |
| Example 13 | Red iron oxide | 1.0 | 0.5 | 141 |
| Comparative Example 3 | Red iron oxide | 1.0 | - | 121 |
| Comparative Example 7 | Red iron oxide | - | 2.0 | 133 |
| Example 4 | Black iron oxide | 1.0 | 0.5 | 133 |
| Example 10 | Black iron oxide | 1.0 | 0.5 | 136 |
| Example 14 | Black iron oxide | 1.0 | 0.5 | 140 |
| Comparative Example 4 | Black iron oxide | 1.0 | - | 131 |
| Comparative Example 8 | Black iron oxide | - | 2.0 | 136 |
| Comparative Example 9 | Titanium oxide | - | - | Not measurable |

As shown in Table 1, in the powder material for a cosmetic of Comparative Example 9 (not treated), the water droplet penetrates into the powder material for a cosmetic to disappear on the surface thereof, and therefore, there was no contact angle. The powder materials for a cosmetic of Examples 1 to 4 tended to exhibit more excellent water repellency than that of the powder materials for a cosmetic of Comparative Examples 1 to 4. The powder materials for a cosmetic of Examples 11 to 14 tended to exhibit more excellent water repellency than that of the powder materials for a cosmetic of Comparative Examples 1 to 8. Therefore, it can be said that the powder material for a cosmetic according to the present invention, which is surface-treated with a combination of a MEL (the component (a)) and DG4ISA (the component (b)), has higher water resistance than that of a powder material for a cosmetic that is covered with only one of a MEL and DG4ISA. In addition, the powder materials for a cosmetic of Examples 7 to 10 (and as described above, Examples 11 to 14) exhibited more excellent water repellency than that of the powder materials for a cosmetic of Comparative Examples 1 to 4. Therefore, it can be said that high water resistance can be obtained even using water instead of an organic solvent in preparation of the liquid mixture.

### [Oil agent dispersibility assessment test]

It is known that the viscosity of a dispersion in which a powder material for a cosmetic is dispersed in an oil agent decreases with an increase in the dispersibility of the powder material for a cosmetic in the oil agent. Therefore, in an oil agent dispersibility assessment test, the viscosities of dispersions in which the powder materials for a cosmetic of Examples 1, 7, and 11, and Comparative Examples 1 and 5 were dispersed in an oil agent were measured using a B-type viscometer. Each dispersion was prepared by stirring and mixing 60 g of a powder material for a cosmetic and 40 g of an oil agent at 1000 rpm for 10 minutes. As the oil agent, glyceryl tri(caprylate/caprate), which is an ester oil agent, squalene, which is a hydrocarbon oil agent, and dimethicone oil, which is a silicone oil agent, were used. The results are shown in FIG. 1.

FIG. 1 is a graph showing results of the oil agent dispersibility assessment test. The viscosities of dispersions prepared using Examples 1, 7, and 11, which are a powder material for a cosmetic the surface of which is covered (surface-treated) with a MEL and DG4ISA, and Comparative Example 5, which is a powder material for a cosmetic the surface of which is only covered (surface-treated) with DG4ISA, were lower than the viscosity of a dispersion prepared using Comparative Example 1, which is a powder material for a cosmetic the surface of which is only covered (surface-treated) with a MEL. In addition, the viscosity of a dispersion prepared using Example 11 was lower than that of a dispersion prepared using Comparative Example 5. Thus, it can be said that the powder material for a cosmetic according to the present invention, which is surface-treated with a combination of a MEL (the component (a)) and DG4ISA (the component (b)), has a dispersibility that is equal to or higher than that of DG4ISA, and has more excellent dispersibility in an oil agent than that of a powder material for a cosmetic that is only covered with a MEL. In addition, the powder materials for a cosmetic of Examples 1, 7, and 11 had similar dispersibilities in an oil agent. Therefore, it can be said that there is not a large difference in dispersibility in an oil agent between a powder material for a cosmetic prepared using a liquid mixture containing an organic solvent and that which is prepared using a liquid mixture containing water.

### [Emulsion stability assessment]

It is known that MEL-covered pigments can form a Pickering emulsion, which has high emulsion stability, without use of a surfactant. In order to confirm that such a characteristic can be maintained even in the case in which the combination treatment is performed using a MEL in combination with an ester compound, the following emulsion stability assessment was conducted using the powder materials for a cosmetic of Examples 1, 5 to 7, and 11 and Comparative Examples 1, 5, and 10 to 12. Initially, 5 g of an alkane having 9 to 12 carbon atoms (C9 to C12 alkanes) and 0.5 g of a powder material for a cosmetic were put into a UM sample bottle (50 mL), and mixed by manually shaking the bottle. Thereafter, 5 g of ion-exchanged water was added, followed by further mixing by shaking. The UM sample bottle was allowed to stand. The appearance of the dispersion in the UM sample bottle was observed after 1 minute, 30 minutes, and 1 hour, and assessed on a rating scale of 1 to 4 (four levels) according to assessment criteria described below. The higher the value, the higher the rating. A rating of 4 indicates sufficient emulsion stability. The results are shown in Table 2.

### (Assessment criteria)

4: The emulsified state is maintained 1 hour after the bottle is allowed to stand (good).
3: Demulsification occurs within 1 hour after the bottle is allowed to stand (fair).
2: Demulsification occurs within 30 minutes after the bottle is allowed to stand (bad).
1: Demulsification occurs within 1 minute after the bottle is allowed to stand (very bad).

**[Table 2]**

| | Powder for cosmetic | MEL content (mass%) | DG4ISA content (mass%) | Emulsion stability (rating) |
|---|---|---|---|---|
| Example 1 | Titanium oxide | 1.0 | 0.5 | 4 |
| Example 7 | Titanium oxide | 1.0 | 0.5 | 4 |
| Example 11 | Titanium oxide | 1.0 | 0.5 | 4 |
| Comparative Example 1 | Titanium oxide | 1.0 | - | 4 |
| Comparative Example 5 | Titanium oxide | - | 2.0 | 1 |
| Example 5 | Titanium oxide | 0.5 | 0.1 | 4 |
| Comparative Example 10 | Titanium oxide | 0.3 | 0.1 | 3 |
| Example 6 | Titanium oxide | 3.0 | 10.0 | 4 |
| Comparative Example 11 | Titanium oxide | 4.0 | 10.0 | 3 |
| Comparative Example 12 | Titanium oxide | 3.0 | 11.0 | 2 |

As shown in Table 2, the powder materials for a cosmetic of Examples 1, 7, 11, 5, and 6 and Comparative Example 1 had an emulsion stability rating of 4, which indicates good emulsion stability. Therefore, it can be said that the powder material for a cosmetic according to the present invention has excellent emulsion stability as with a powder material for a cosmetic that is only covered with a MEL. Meanwhile, the powder materials for a cosmetic of Comparative Examples 5 and 10 to 12 had an emulsion stability rating of at most 3, which indicates insufficient emulsion stability.

### [Example 15, Comparative Example 13, and Comparative Example 14]

Cosmetics (W/O type liquid foundations) according to Example 15, Comparative Example 13, and Comparative Example 14, which had compositions shown in Table 3, were prepared by a production method described below. It should be noted that the content of each component is expressed in mass%.

### (Method for producing W/O type liquid foundation)

Initially, components A that are oil components were stirred and mixed thoroughly using a disper until a homogeneous mixture was obtained. Components B that are powder components were gradually added to the mixture of the components A, which was kept stirred using the disper, followed by thorough stirring and mixing until a homogeneous mixture was obtained. Components C that are aqueous components were mixed together. Thereafter, the mixture of the components C was gradually added to the mixture of the components A and B, which was kept stirred using the disper, to obtain an emulsion.

**[Table 3]**

| | | Contents | | |
|---|---|---|---|---|
| | | Example 15 | Comparative Example 13 | Comparative Example 14 |
| Components A | MAKIBASE SEB | 2.0 | 2.0 | 2.0 |
| | Coco-caprylate/caprate | 10.3 | 10.3 | 10.3 |
| | Tetraalkylammonium hectorite | 1.0 | 1.0 | 1.0 |
| | Ethanol | 0.6 | 0.6 | 0.6 |
| | C9 to C12 alkanes | 15.0 | 15.0 | 15.0 |
| | Plant oil | 2.0 | 2.0 | 2.0 |
| Components B | Powder material for cosmetic of Example 1 (titanium oxide) | 8.5 | - | - |
| | Powder material for cosmetic of Example 2 (yellow iron oxide) | 1.0 | - | - |
| | Powder material for cosmetic of Example 3 (red iron oxide) | 0.3 | - | - |
| | Powder material for cosmetic of Example 4 (black iron oxide) | 0.2 | - | - |
| | Powder material for cosmetic of Comparative Example 1 (titanium oxide) | - | 8.5 | - |
| | Powder material for cosmetic of Comparative Example 2 (yellow iron oxide) | - | 1.0 | - |
| | Powder material for cosmetic of Comparative Example 3 (red iron oxide) | - | 0.3 | - |
| | Powder material for cosmetic of Comparative Example 4 (black iron oxide) | - | 0.2 | - |
| | Powder material for cosmetic of Comparative Example 5 (titanium oxide) | - | - | 8.5 |
| | Powder material for cosmetic of Comparative Example 6 (yellow iron oxide) | - | - | 1.0 |
| | Powder material for cosmetic of Comparative Example 7 (red iron oxide) | - | - | 0.3 |
| | Powder material for cosmetic of Comparative Example 8 (black iron oxide) | - | - | 0.2 |
| | DG4ISA-surface-treated talc | 5.0 | 5.0 | 5.0 |
| Components C | Purified water | Balance | Balance | Balance |
| | 1,3-butyleneglycol | 5.0 | 5.0 | 5.0 |
| | Magnesium sulfate heptahydrate | 1.0 | 1.0 | 1.0 |

The W/O type liquid foundations obtained in Example 15, Comparative Example 13, and Comparative Example 14 were assessed in terms of dispersibility observed using a microscope and feel to the skin when applied thereto. The feel to the skin was assessed in terms of feeling of weight, adhesion, and feeling of use. The adhesion and feeling of use were assessed in four levels according to the following assessment criteria. The results are shown in Table 4.

### (Assessment criteria)

### · Adhesion (long-lastingness)

A: very good
B: good
C: bad
D: very bad

### · Feeling of use

A: very good
B: good
C: bad
D: very bad

**[Table 4]**

| | Surface treatment | Dispersibility | Feel to skin | | |
|---|---|---|---|---|---|
| | | | Feeling of weight | Adesion | Feeling of use |
| Example 15 | Combination (MEL+DG4ISA) | Homogeneous | Moderate | A | A |
| Comparative Example 13 | MEL | Aggregated | Moderate | A | B |
| Comparative Example 14 | DG4ISA | Homogeneous | Light | C | B |

As a result of the dispersibility assessment, the aggregation of the powder was observed in the cosmetic of Comparative Example 13. Meanwhile, the powder was uniformly dispersed in the cosmetics of Example 15 and Comparative Example 14. It was confirmed that the cosmetic according to the present invention has as excellent dispersibility as that of a cosmetic that is only surface-treated with DG4ISA.

As a result of the feel assessment, the cosmetic of Comparative Example 14 was lightweight and less adhesive. Meanwhile, the cosmetics of Comparative Example 13 and Example 15 both exhibited an excellent result in every assessment item. It was confirmed that the cosmetic according to the present invention has excellent feel that is not obtained by a cosmetic that is only surface-treated with DG4ISA.

### [Example 16, Comparative Example 15, and Comparative Example 16]

Cosmetics (W/O type liquid foundations) according to Example 16, Comparative Example 15, and Comparative Example 16, which had compositions shown in Table 5, were prepared by a production method described below. It should be noted that the content of each component is expressed in mass%.

### (Method for producing W/O type liquid foundation)

Initially, components A (a component A1 and a component A2) that are oil components were stirred and mixed thoroughly using a disper until a homogeneous mixture was obtained. A component B that is a powder component was gradually added to the mixture of the components A, which was kept stirred using the disper, followed by thorough stirring and mixing until a homogeneous mixture was obtained. Components C (a component C1 and a component C2) that are aqueous components were mixed together. Thereafter, the mixture of the components C was gradually added to the mixture of the components A and B, which was kept stirred using the disper, to obtain an emulsion.

**[Table 5]**

| | | | Contents | | |
|---|---|---|---|---|---|
| | | | Example 16 | Comparative Example 15 | Comparative Example 16 |
| Components A | Components A1 | Cyclopentasiloxane | 7.0 | 7.0 | 7.0 |
| | | Methylphenil polysiloxane | 2.0 | 2.0 | 2.0 |
| | | Liquid paraffin | 10.0 | 10.0 | 10.0 |
| | | Tetraalkylammonium hectorite | 1.0 | 1.0 | 1.0 |
| | | Propylene carbonate | 0.5 | 0.5 | 0.5 |
| | | Cetyl alcohol | 3.0 | 3.0 | 3.0 |
| | | Vaseline | 2.0 | 2.0 | 2.0 |
| | Components A2 | 2-Methylhexyl para-methoxycinnamate | 5.0 | 5.0 | 5.0 |
| | | Sorbitan monoisostearate | 4.0 | 4.0 | 4.0 |
| Components B | | Powder material for cosmetic of Example 1 (titanium oxide) | 12.7 | - | - |
| | | Powder material for cosmetic of Example 2 (yellow iron oxide) | 1.5 | - | - |
| | | Powder material for cosmetic of Example 3 (red iron oxide) | 0.5 | - | - |
| | | Powder material for cosmetic of Example 4 (black iron oxide) | 0.3 | - | - |
| | | Powder material for cosmetic of Comparative Example 1 (titanium oxide) | - | 12.7 | - |
| | | Powder material for cosmetic of Comparative Example 2 (yellow iron oxide) | - | 1.5 | - |
| | | Powder material for cosmetic of Comparative Example 3 (red iron oxide) | - | 0.5 | - |
| | | Powder material for cosmetic of Comparative Example 4 (black iron oxide) | - | 0.3 | - |
| | | Powder material for cosmetic of Comparative Example 5 (titanium oxide) | - | - | 12.7 |
| | | Powder material for cosmetic of Comparative Example 6 (yellow iron oxide) | - | - | 1.5 |
| | | Powder material for cosmetic of Comparative Example 7 (red iron oxide) | - | - | 0.5 |
| | | Powder material for cosmetic of Comparative Example 8 (black iron oxide) | - | - | 0.3 |
| | | MEL-surface-treated mica | 5.0 | 5.0 | 5.0 |
| | | Spherical cellulose beads | 3.0 | 3.0 | 3.0 |
| | | Amino acid-surface-treated zinc oxide | 3.0 | 3.0 | 3.0 |
| Components C | Components C1 | Purified water | 16.7 | 16.7 | 16.7 |
| | | 1,3-butyleneglycol | 5.0 | 5.0 | 5.0 |
| | | Glycerol | 5.0 | 5.0 | 5.0 |
| | | Sodium chloride | 0.5 | 0.5 | 0.5 |
| | | Antiseptic | 0.3 | 0.3 | 0.3 |
| | | Aqueous Aphanothece sacrum polysaccharide BG solution | 2.0 | 2.0 | 2.0 |
| | Components C2 | Purified water | 9.9 | 9.9 | 9.9 |
| | | Hyaluronic acid | 0.1 | 0.1 | 0.1 |

The W/O type cream foundations obtained in Example 16, Comparative Example 15, and Comparative Example 16 were assessed in terms of dispersibility observed using a microscope and feel to the skin when applied thereto, in a manner similar to that described above. The results are shown in Table 6.

**[Table 6]**

| | Surface treatment | Dispersibility | Feel to skin | | |
|---|---|---|---|---|---|
| | | | Feeling of weight | Adesion | Feeling of use |
| Example 16 | Combination (MEL+DG4ISA) | Homogeneous | Moderate | A | A |
| Comparative Example 15 | MEL | Aggregated | Moderate | A | C |
| Comparative Example 16 | DG4ISA | Homogeneous | Heavy | C | D |

As a result of the dispersibility assessment, the aggregation of the powder was observed in the cosmetic of Comparative Example 15. Meanwhile, the powder was uniformly dispersed in the cosmetics of Example 16 and Comparative Example 16. It was confirmed that the cosmetic according to the present invention has as excellent dispersibility as that of a cosmetic that is only surface-treated with DG4ISA.

As a result of the feel assessment, the cosmetic of Comparative Example 16 was heavy and had poor feeling of use. Meanwhile, the cosmetic of Example 16 exhibited an excellent result in every assessment item. It was confirmed that the cosmetic according to the present invention has excellent feel that is not obtained by a cosmetic that is only surface-treated with a MEL or DG4ISA.

### [Example 17, Comparative Example 17, and Comparative Example 18]

Cosmetics (W/O type concealers) according to Example 17, Comparative Example 17, and Comparative Example 18, which had compositions shown in Table 7, were prepared by a production method described below. It should be noted that the content of each component is expressed in mass%.

### (Method for producing W/O type concealer)

Initially, components A that are oil components were stirred and mixed thoroughly using a disper until a homogeneous mixture was obtained. A component B that is a powder component was gradually added to the mixture of the components A, which was kept stirred using the disper, followed by thorough stirring and mixing until a homogeneous mixture was obtained. Components C that are aqueous components were mixed together. Thereafter, the mixture of the components C was gradually added to the mixture of the components A and B, which was kept stirred using the disper, to obtain an emulsion.

**[Table 7]**

| | | Contents | | |
|---|---|---|---|---|
| | | Example 17 | Comparative Example 17 | Comparative Example 18 |
| Components A | Stearic acid | 2.5 | 2.5 | 2.5 |
| | Self-emulsifying glycerol monostearate | 1.8 | 1.8 | 1.8 |
| | Cetyl alcohol | 0.5 | 0.5 | 0.5 |
| | Olive squalane | 9.9 | 9.9 | 9.9 |
| Components B | Powder material for cosmetic of Example 1 (titanium oxide) | 17.0 | - | - |
| | Powder material for cosmetic of Example 2 (yellow iron oxide) | 2.0 | - | - |
| | Powder material for cosmetic of Example 3 (red iron oxide) | 0.6 | - | - |
| | Powder material for cosmetic of Example 4 (black iron oxide) | 0.4 | - | - |
| | Powder material for cosmetic of Comparative Example 1 (titanium oxide) | - | 17.0 | - |
| | Powder material for cosmetic of Comparative Example 2 (yellow iron oxide) | - | 2.0 | - |
| | Powder material for cosmetic of Comparative Example 3 (red iron oxide) | - | 0.6 | - |
| | Powder material for cosmetic of Comparative Example 4 (black iron oxide) | - | 0.4 | - |
| | Powder material for cosmetic of Comparative Example 5 (titanium oxide) | - | - | 17.0 |
| | Powder material for cosmetic of Comparative Example 6 (yellow iron oxide) | - | - | 2.0 |
| | Powder material for cosmetic of Comparative Example 7 (red iron oxide) | - | - | 0.6 |
| | Powder material for cosmetic of Comparative Example 8 (black iron oxide) | - | - | 0.4 |
| | Spherical cellulose beads | 3.0 | 3.0 | 3.0 |
| Components C | Purified water | Balance | Balance | Balance |
| | 1,3-butyleneglycol | 9.0 | 9.0 | 9.0 |
| | Antiseptic | 0.3 | 0.3 | 0.3 |
| | 2-Amino-2-methyl-1,3-proponediol | 0.5 | 0.5 | 0.5 |
| | Xanthan gum | 0.4 | 0.4 | 0.4 |

The W/O type concealers obtained in Example 17, Comparative Example 17, and Comparative Example 18 were assessed in terms of dispersibility observed using a microscope, in a manner similar to that described above. These concealers were also assessed in terms of feel to the skin when applied thereto. The feel to the skin was assessed in terms of feeling of weight, adhesion, and feeling of use, in a manner similar to that described above. The feel to the skin was also assessed in terms of spreadability, which was assessed in four levels according to the following assessment criteria. The results are shown in Table 8.

### (Assessment criteria)

### · Spreadability

A: very good
B: good
C: bad
D: very bad

**[Table 8]**

| | Surface treatment | Dispersibility | Feel to skin | | | |
|---|---|---|---|---|---|---|
| | | | Feeling of weight | Adesion | Spreadability | Feeling of use |
| Example 17 | Combination (MEL+DG4ISA) | Homogeneous | Moderate | A | A | A |
| Comparative Example 17 | MEL | Aggregated | Heavy | A | A | B |
| Comparative Example 18 | DG4ISA | Homogeneous | Moderate | C | D | C |

As a result of the dispersibility assessment, the aggregation of the powder was observed in the cosmetic of Comparative Example 17. Meanwhile, the powder was uniformly dispersed in the cosmetics of Example 17 and Comparative Example 18. It was confirmed that the cosmetic according to the present invention has as excellent dispersibility as that of a cosmetic that is only surface-treated with DG4ISA.

As a result of the feel assessment, the cosmetic of Comparative Example 17 was heavy, and the cosmetic of Comparative Example 18 had poor adhesion, spredability, and feeling of use. Meanwhile, the cosmetic of Example 17 exhibited an excellent result in every assessment item. It was confirmed that the cosmetic according to the present invention has excellent feel that is not obtained by a cosmetic that is only surface-treated with a MEL or DG4ISA.

### INDUSTRIAL APPLICABILITY

The powder material for a cosmetic of the present invention is suitable for cosmetics and the like, and particularly, suitable for use in make-up cosmetics containing a pigment, such as foundation, eye shadow, eyebrow liner, and blush.

## Claims

1. A powder material for a cosmetic, comprising a powder for a cosmetic surface-treated with a surface treatment agent,
wherein
the surface treatment agent contains
a component (a): a mannosylerythritol lipid represented by 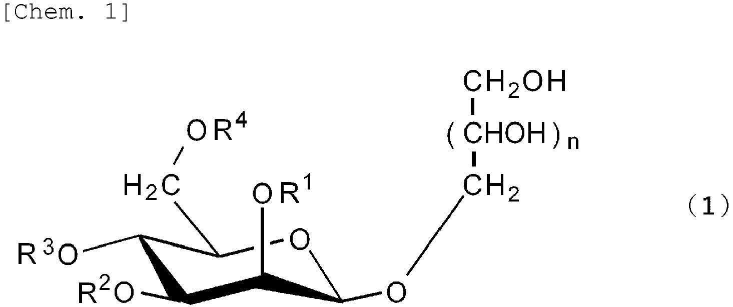
(in the formula (1), R¹ and R² each independently represent an aliphatic acyl group having 6 to 20 carbon atoms, R³ and R⁴ each independently represent a hydrogen atom or an acetyl group, and n represents an integer of 2 to 4), and
a component (b): an ester compound of glycerol and a fatty acid having 8 to 20 carbon atoms,
the content of the component (a) is 0.5 to 3 mass%, and
the content of the component (b) is 0.1 to 10 mass%.

2. The powder material for a cosmetic according to claim 1, wherein
the component (b) is selected from the group consisting of diglyceryl tetraisostearate, diglyceryl triisostearate, glyceryl tri(caprylate/caprate), glyceryl dilaurate, glyceryl triisostearate, decaglyceryl monoisostearate, and decaglyceryl diisostearate.

3. The powder material for a cosmetic according to claim 1, wherein
R¹ and R² each independently represent an aliphatic acyl group having 10 to 14 carbon atoms.

4. The powder material for a cosmetic according to claim 1, wherein
the powder for a cosmetic is at least one selected from the group consisting of titanium oxide, yellow iron oxide, red iron oxide, black iron oxide, talc, zinc oxide, silicon oxide, pearl mica, mica, and sericite.

5. A method for producing a powder material for a cosmetic, comprising
mixing a powder for a cosmetic with a liquid mixture obtained by dispersing, suspending, or dissolving, in a solvent,
a component (a): a mannosylerythritol lipid represented by 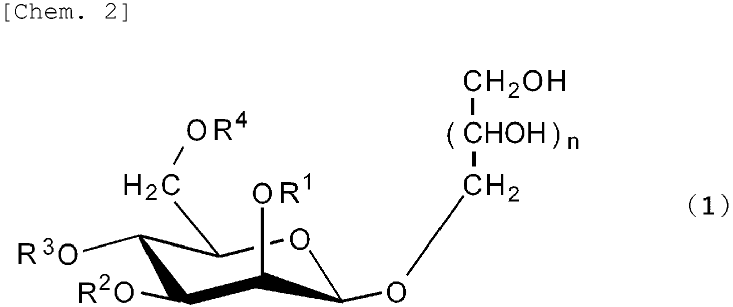
(in the formula (1), R¹ and R² each independently represent an aliphatic acyl group having 6 to 20 carbon atoms, R³ and R⁴ each independently represent a hydrogen atom or an acetyl group, and n represents an integer of 2 to 4), and
a component (b): an ester compound of glycerol and a fatty acid having 8 to 20 carbon atoms, and
heat-treating the mixture obtained by the mixing.

6. The method for producing a powder material for a cosmetic according to claim 5, wherein
in the mixing, the mixing ratio by mass of the solvent to the component (a) contained in the liquid mixture is adjusted to at least 10:1.

7. The method for producing a powder material for a cosmetic according to claim 5 or 6, wherein
in the heat-treating, the mixture is heat-treated at 70 to 150°C.

8. The method for producing a powder material for a cosmetic according to claim 5 or 6, wherein
the solvent is an organic solvent and/or water.

9. A cosmetic comprising the powder material for a cosmetic according to any one of claims 1 to 4.
